# EUROPÄISCHE PATENTANMELDUNG

(11) **EP 0 688 573 A1**
(43) Veröffentlichungstag der Anmeldung: **27.12.1995**
(21) Anmeldenummer: 94104555.1
(22) Anmeldetag: 23.03.1994
(51) Int. Cl.: A61M 16/00

(54) **Vorrichtung zur Durchführung einer Mund-zu-Mund-Beatmung**

(71) Anmelder: TOHA-PLAST GmbH, D-37079 Göttingen (DE)
(72) Erfinder: Hackel, Thomas, D-37079 Göttingen (DE)
(74) Vertreter: Rehberg, Elmar, Dipl.-Ing.

(57) **Zusammenfassung**

Die Vorrichtung für die Durchführung einer Mund-zu-Mund-Beatmung weist einen rohrartigen Grundkörper 1 auf, an dessen beiden Enden je ein Mundstück 10, 12 für den Helfer einerseits und den Patienten andererseits angeordnet sind. Im Durchgangsquerschnitt 6 des Grundkörpers 1 ist ein hydrophober Tiefenfilter 2 angeordnet. Der rohrartige Grundkörper 1 ist mit einem sich im wesentlichen radial erstreckenden Zuschnitt 3 aus einem durchsichtigen Flächengebilde versehen.

## Beschreibung

Die Erfindung bezieht sich auf eine Vorrichtung für die Durchführung einer Mund-zu-Mund-Beatmung mit einem rohrartigen Grundkörper, der an seinen beiden Enden je ein Mundstück für den Helfer einerseits und den Patienten andererseits, sowie eine das Eindringen des Mundstücks in den Mund des Patienten begrenzende Mundplatte aufweist und in dessen Durchgangsquerschnitt ein hydrophober Tiefenfilter angeordnet ist. Eine solche Vorrichtung wird als Beatmungshilfe zwischen Helfer und Patient eingesetzt, insbesondere nach Verkehrsunfällen, bei Herzstillstand und bei Aufrechterhaltung der Lebensfunktionen. Er kann von Helfern der verschiedensten Art, beispielsweise von Ärzten, Polizei, Feuerwehr, aber auch von Laienhelfern sinnvoll genutzt werden. Er dient der Herz-Lungen-Wiederbelebung und kann z. B. auch bei Kreislaufnotfällen eingesetzt werden.

Eine Vorrichtung der eingangs beschriebenen Art ist aus der DE 38 37 433 A1 bekannt. Die Vorrichtung wird als "Göttinger Tubus" bezeichnet. Die Vorrichtung weist einen zweiteiligen rohrartigen Grundkörper auf, der eine radiale Erweiterung besitzt, wobei im Bereich dieser Erweiterung eine Trennfuge vorgesehen ist. Gleichzeitig ist an dieser Stelle im Durchgangsquerschnitt ein hydrophober Tiefenfilter untergebracht, durch den die Luft bei der Mund-zu-Mund-Beatmung fließt. Dieser hydrophobe Tiefenfilter schützt Helfer und Patient vor gegenseitiger Ansteckung. Zu diesem rohrartigen Grundkörper gehört weiterhin ein Verlängerungskörper, der ebenfalls rohrartig ausgebildet ist und der eine gekrümmte Mundplatte trägt, die dem Mund des Patienten zugeordnet ist. Der Verlängerungskörper kann mit dem Grundkörper über einen Anschlußkonus zusammengesteckt werden. Der Verlängerungskörper setzt sich in einen Guedel fort, also einen gebogenen rohrartigen Körper, der patientenseitig vor der Mundplatte am Verlängerungskörper angeordnet ist. Dieser Guedel dient dazu, die Zunge des Patienten in eine solche Lage zu bringen und darin zu halten, daß sie die Luftröhre des Patienten nicht verschließt und den Zungengrund in eine stabile Lage versetzt. Beim Einführen des Guedels in den Mund des bewußtlosen Patienten ist es erforderlich, den Verlängerungskörper um 180° um seine Achse im Mund des Patienten zu drehen, um durch diesen Drehvorgang die Zunge in die vorgesehene Relativlage zu bringen. Diese Handhabung muß sachgerecht ausgeführt werden, um einer Verletzungsgefahr für den Patienten entgegenzuwirken. Demzufolgen gehört zur Anwendung der bekannten Vorrichtung eine entsprechende Ausbildung und Übung bei der Anwendung, so daß die Vorrichtung praktisch nur von Fachleuten und ausgebildeten Laienhelfern nutzbar ist. Die bekannte Vorrichtung hat den weiteren Nachteil, daß ihr Einsatz und ihre Anwendung an bei Bewußtsein befindlichen Übungspersonen nicht geübt werden kann; die vorgesehene Relativlage des Guedels zur Zunge der Übungsperson führt unweigerlich zu einem nicht unterdrückbaren Brechreiz. Weiterhin ist an der bekannten Vorrichtung nachteilig, daß eine Verstopfungsgefahr besteht, insbesondere dann, wenn nach einem Erbrechungsvorgang des Patienten Speisereste in den Querschnitt der Vorrichtung gelangen. Die bekannte Vorrichtung bietet auch keinen Schutz vor gegenseitiger Ansteckung zwischen Helfer und Patient durch Blut oder Speichel. Insbesonder nach Verkehrsunfällen, bei denen der Patient im Bereich des Kopfes, insbesondere Gesicht oder Ohren, blutet, ist ein Ansteckungsschutz von besonderer Bedeutung, insbesondere gegen Aids oder Hepatitis B.

Aus dem DE-GM 87 14 617 ist eine Vorrichtung bekannt, die als Vorläufer der gattungsgemäßen Vorrichtung angesehen werden kann. Die Kunststofformteile sind ähnlich ausgebildet. Es ist auch hier ein rohrartiger Grundkörper und ein rohrartiger Verlängerungskörper mit fester Mundplatte und Guedel vorgesehen. Infolge des hier noch fehlenden hydrophoben Tiefenfilters ist die direkte Ansteckungsgefahr zwischen Helfer und Patient in besonders nachteiliger Weise gegeben.

Weiterhin sind Masken für die Mund-zu-Mund-Beatmung bekannt. Da die Maske an die Gesichtsform des Patienten angepaßt sein soll, gibt es verschiedene Größen. Die Maske besteht aus transparentem Material und weist ein Mundstück sowie ein Rückschlagventil auf, welches so eingebaut ist, daß die Atemluft vom Helfer zum Patienten durchgelassen, die entgegengesetzte Richtung jedoch blockiert wird. Es sind separate Öffnungen für das Ausatmen der Atemluft durch den Patienten vorgesehen. Damit tritt aber nur ein gewisser Schutz für den Helfer, nicht jedoch für den Patienten ein. Ein Schutz vor gegenseitiger Ansteckung durch Blut oder Speichel ist nicht gegeben.

Aus dem DE 88 01 386 U1 ist eine Schutzfolie zum Bedecken des Gesichtes eines Patienten bei der Mund-zu-Mund-Beatmung bekannt. Dabei wird ein Zuschnitt eines Flächengebildes aus Kunststoffolie benutzt, welcher eine das Gesicht des Patienten überdeckende Größe aufweist. Etwa mittig in der Schutzfolie ist eine Öffnung vorgesehen, die durch einen Filter abgedeckt wird. Bei der Anwendung dieser Schutzfolie ist keineswegs sichergestellt, daß die von dem Helfer ausgeblasene Luft auch überhaupt in die Lunge des Patienten kommt.

Der Erfindung liegt die Aufgabe zugrunde, eine Vorrichtung der eingangs beschriebenen Art derart weiterzubilden, daß Helfer und Patient wirksamer als bisher vor gegenseitiger Ansteckung geschützt sind. Trotzdem soll die Vorrichtung vergleichsweise einfacher aufgebaut sein, so daß sie auch von nicht-geschulten Helfern wirksam eingesetzt werden kann, wobei nicht die Gefahr besteht, daß erbrochene Speisereste die Beatmung verhindern.

Erfindungsgemäß wird dies bei einer Vorrichtung der eingangs beschriebenen Art dadurch erreicht, daß der rohrartige Grundkörper mit einem sich im wesentlichen radial zu dem Grundkörper erstreckenden Zuschnitt aus einem durchsichtigem Flächengebilde aus Kunststoffolie, textilem oder papierähnlichem Material versehen ist, daß der Zuschnitt aus durchsichtigem Flächengebilde eine zumindest das Gesicht des Patienten überdeckende Größe aufweist, und daß das patientenseitige Mundstück mit mindestens einem den freien Querschnitt durchsetzenden Steg versehen ist.

Die Erfindung geht damit von einer Vorrichtung aus, die bereits einen hydrophoben Tiefenfilter im Durchgangsquerschnitt, durch den die Luft einwärts und auswärts mit Bezug auf den Patienten fließt, ausgestattet ist. Damit ist zunächst einmal ein beidseitiger Schutz des Helfers und des Patienten in diesem Bereich gegeben. Durch den Zuschnitt aus einem durchsichtigen Flächengebilde, der dichtend und abdeckend an den rohrartigen Grundkörper anschließt, ist es möglich, zumindest das Gesicht, zweckmäßig jedoch den gesamten Kopf des Patienten abzudecken, so daß der Helfer weder mit Blut noch mit Speichel des Patienten in Berührung kommen kann. Der Zuschnitt kann durchaus runde, ovale, rechteckige oder auch rhombenartige Form aufweisen und besitzt eine im Vergleich zum Grundkörper beachtliche Größe, läßt sich aber andererseits auf kleinstem Raum unterbringen, so daß insoweit die Handhabbarkeit nicht beeinträchtigt ist. Die Vorrichtung kann vorteilhaft damit auch auf kleinem Raum untergebracht und mitgeführt werden, z. B. im Verbandskasten eines PKW. Durch den bewußten Verzicht auf den Guedel wird die Vorrichtung auch für Laienhelfer anwendbar; die Einführung des patientenseitigen Mundstückes in den Mund des Patienten ist einfach durchführbar und auch für solche staatlichen Rechtssysteme tolerierbar, die an sich das Eindringen in den Mund eines Patienten, z. B. mit einem Guedel, dem Arzt vorbehalten.

Ein weiterer Vorteil der neuen Vorrichtung besteht darin, daß sie ohne weiteres auch an Übungspuppen und Übungspersonen mit Bewußtsein anwendbar ist und damit eine vorsorgliche Handhabung zu Ausbildungszwecken gestattet. Die vergleichsweise einfache Ausbildung der Vorrichtung steht im Gegensatz zu der damit erreichten gegenseitigen Schutzfunktion. Die Anwendung von Rückschlagventilen und Gesichtsmasken wird überflüssig. Die Verwendung von einem durchsichtigen Flächengebilde für den Zuschnitt ist insofern sinnvoll, als dadurch die Sicht auf das Gesicht des Patienten nicht beeinträchtigt wird. Die Atemfunktion und die unter Umständen vorhandene Verletzung des Patienten im Gesicht können auch während der Anwendung der Vorrichtung überwacht und kontrolliert werden. Das durchsichtige Flächengebilde kann aus Kunststoffolie, aus einem textilen oder auch papierähnlichen Material bestehen.

Der Zuschnitt aus durchsichtiger Kunststoffolie sollte mindestens eine das Gesicht des Patienten überdeckende Größe aufweisen. Es empfielt sich jedoch eine noch größere Gestaltung, so daß auch die Ohren sowie weitere Kopfpartien des Patienten überdeckt werden können. Der Patient kann schließlich bei einem Unfall an den verschiedensten Stellen des Kopfes verletzt sein und z. B. bluten.

Das patientenseitige Mundstück am Grundkörper oder am Verlängerungskörper ist mit mindestens einem den freien Querschnitt durchsetzenden Steg versehen. Die Stege können parallel zueinander oder auch gitterartig angeordnet sein. Sie haben die Aufgabe, das Eindringen von erbrochenen Speiseresten in den Querschnitt der Vorrichtung und dadurch bewirkte Verstopfungen zu verhindern. Solche Speisereste können von den Stegen durch Abschütteln oder bei einem beginnenden Luftstoß der Beatmung durch den Helfer leicht entfernt werden. Ebenso kann hier ein Vorfilter als Grobfilter zusätzlich angebracht werden.

Der Zuschnitt aus dem durchsichtigen Flächengebilde kann mit dem Grundkörper unlösbar verbunden sein, wodurch zugleich eine besonders gute Abdichtung erreicht wird. Hier kommt insbesondere eine Verklebung oder Verschweißung in Betracht, die entweder nachträglich nach der Herstellung des Grundkörpers als Kunststoffspritzteil oder Kunststoffspritzteile erfolgen kann. Es ist auch möglich, die Verschweißung oder Verklebung mit dem Grundkörper zugleich mit der Montage der Teile des Grundkörpers durchzuführen.

Andererseits kann der Zuschnitt aus durchsichtiger Kunststoffolie mit dem Grundkörper auch lösbar verbunden sein. Dies muß die Dichtheit nicht beeinträchtigen. Ein wirksamer gegenseitiger Infektionsschutz ist auch hier gegeben.

Der Grundkörper kann aus mehreren Teilen zusammengesetzt sein, wobei der Zuschnitt zweckmäßig zwischen den Teilen des Grundkörpers angeordnet ist. Es ist aber auch möglich, den Zuschnitt gleichsam mundstückseitig außen an den Grundkörper anzusetzen. In der Regel empfielt sich jedoch, den Grundkörper aus mehreren Teilen zusammenzusetzen, schon um die Montage des hydrophoben Tiefenfilters durchzuführen.

In allen Fällen weist der Zuschnitt aus durchsichtiger Kunststoffolie eine an den Grundkörper angepaßte Durchbrechung auf. Die Durchbrechung besitzt zweckmäßig kreisrunde Form und ist auf den Durchmesser an der Anordnungsstelle des Zuschnittes am Grundkörper abgestimmt. Es kann ein rohrartiger Verlängerungskörper vorgesehen sein, der eine sich radial und gekrümmt erstreckende Mundplatte aufweist. Dieser Verlängerungskörper ist an den Grundkörper ansetzbar oder auch einstückig mit diesem oder einem Teil des Grundkörpers herstellbar. Der Verlängerungskörper weist jedoch auch keinen Guedel auf. Er besitzt dann das patientenseitige Mundstück, und die Mundplatte verhindert bzw. begrenzt den Einschiebevorgang des Mundstückes in den Mund des Patienten. Gleichzeitig gestattet es diese Mundplatte, einen gewissen Druck auf den Mund des Patienten, verbunden mit einer Abdichtwirkung, auszuüben, so daß die Atemluft wirksam in den Patienten gelangen kann. Die Mundplatte kann noch zusätzlich mit einer filzartigen Abdeckung versehen werden.

Der Grundkörper und der Verlängerungskörper können über einen Anschlußkonus miteinander verbunden sein, so daß also hier eine lösbare Verbindung zur Anwendung kommt. Damit ergibt sich zugleich auch eine vorteilhafte Anordnungsstelle für den Zuschnitt aus dem durchsichtigen Flächengebilde, der insbesondere lösbar angeordnet werden kann. In Verbindung mit dem Verlängerungskörper ergibt sich der weitere Vorteil, daß der Grundkörper von dem teilweise in den Mund des Patienten hineinreichenden Verlängerungskörper abgenommen werden kann, so daß eine Intubation durch den Verlängerungskörper hindurch erfolgen kann.

Die Erfindung wird anhand mehrerer bevorzugter Ausführungsbeispiele weiter beschrieben und erläutert. Es zeigen:
- Figur 1: einen Querschnitt durch eine erste Ausführungsform der Vorrichtung,
- Figur 2: einen Querschnitt durch eine zweite Ausführungsform der Vorrichtung,
- Figur 3: einen Querschnitt durch eine dritte Ausführungsform der Vorrichtung und
- Figur 4: eine Draufsicht auf den Zuschnitt aus durchsichtiger Kunststoffolie,
- Figur 5: einen Querschnitt durch eine vierte Ausführungsform der Vorrichtung,
- Figur 6: einen Querschnitt durch eine besonders preisgünstig herstellbare Ausführungsform und
- Figur 7: einen Querschnitt durch eine letzte Ausführungsform.

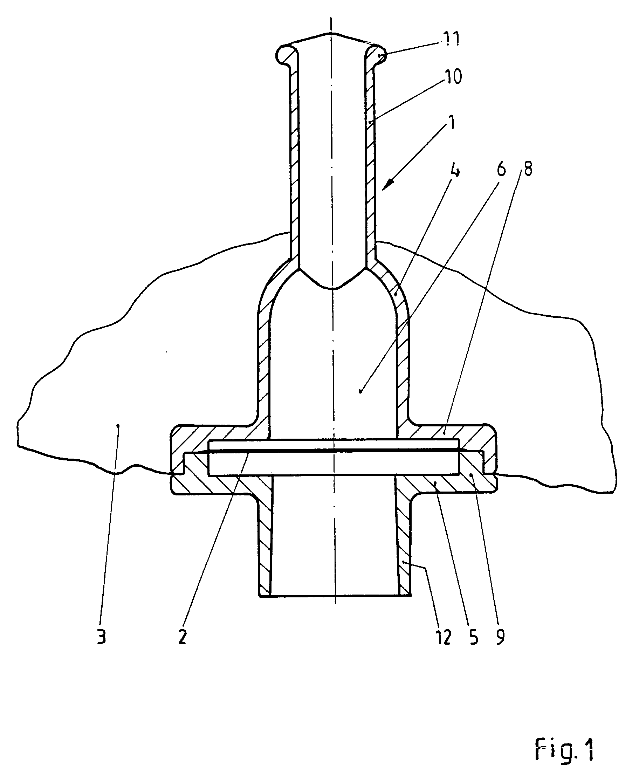

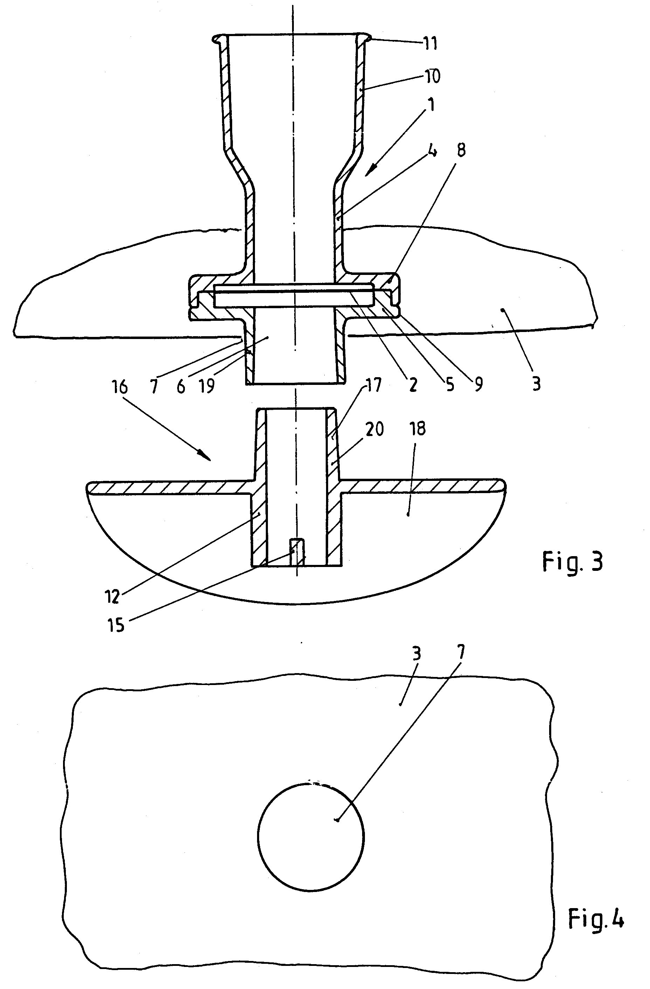

Die Vorrichtung in ihrer Ausführung gemäß Figur 1 weist einen rohrartigen Grundkörper 1 mit einem hydrophoben Tiefenfilter 2 und einen Zuschnitt 3 aus einem durchsichtigen Flächengebilde auf. Der Grundkörper 1 besteht aus einem ersten Formteil 4 aus Kunststoff und einem zweiten Formteil 5, welches ebenfalls als Kunststoffspritzteil ausgebildet ist. In die Trennebene zwischen den beiden Formteilen 4 und 5 ist der hydrophobe Tiefenfilter 2 eingelegt und verankert, so daß von ihm der gesamte Durchgangsquerschnitt 6 des rohrförmigen Grundkörpers 1 abgedeckt wird. Die durch den Durchgangsquerschnitt 6 fließende Luft bei der Mund-zu-Mund-Beatmung - gleich in welcher Richtung - muß somit durch den Tiefenfilter 2 fließen. Damit ist ein wirksamer Schutz vor Infektion für den Helfer und den Patienten gegeben. Der Zuschnitt 3 aus durchsichtigem Flächengebilde ist ebenfalls in die Trennebene zwischen den beiden Formteilen 4 und 5 bei deren Verbindung eingelegt und wird zugleich durch die Verbindung zwischen den Formteilen 4 und 5 unlösbar mit dem Grundkörper 1 verbunden. Es versteht sich, daß der Zuschnitt 3 in seinem Mittelbereich eine Durchbrechung 7 (Figur 4) aufweist, die zweckmäßig kreisrunde Gestalt besitzt und im Durchmesser entsprechend angepaßt ist.

Das Formteil 4 besitzt an seiner Verbindungsstelle zum Formteil 5 eine Erweiterung 8, der eine entsprechende Erweiterung 9 am Formteil 5 zugeordnet ist. Die beiden Erweiterungen 8 und 9 können unter Bildung einer Schnappverbindung mit Wulsten, Rippen o. dgl. (nicht dargestellt) versehen sein. Auch die Zusammenfügung durch eine Klebung oder Schweißung ist möglich. Ansonsten weist das Formteil 4 helferseitig ein Mundstück 10 auf, welches ovale Gestalt (Figur 3) besitzen kann und zweckmäßig einen umlaufenden überstehenden Rand 11 trägt. Das Formteil 5 besitzt patientenseitig ebenfalls ein Mundstück 12, welches runde oder ovale Gestalt besitzen kann. Das Mundstück 12 ist etwas dickwandiger ausgeführt, um einen Beißschutz zu verwirklichen.

Der Zuschnitt 3 aus durchsichtigem Flächengebilde, von dem ein Ausschnitt in Figur 4 in Alleinstellung dargestellt ist, besitzt eine quadratische, rechteckige oder auch runde bzw. ovale Formgebung. Es handelt sich um ein relativ großes Stück eines Flächengebildes, mit dem sich zumindest weite Partien des Gesichtes, insbesondere aber der ganze Kopf abdecken läßt. Die Verwendung von durchsichtigem Flächengebilde gestattet es dennoch, den Verlauf der Beatmung und zusätzlich beim Patienten vorhandene Verletzungen während der Hilfeleistung zu überwachen bzw. zu kontrollieren.

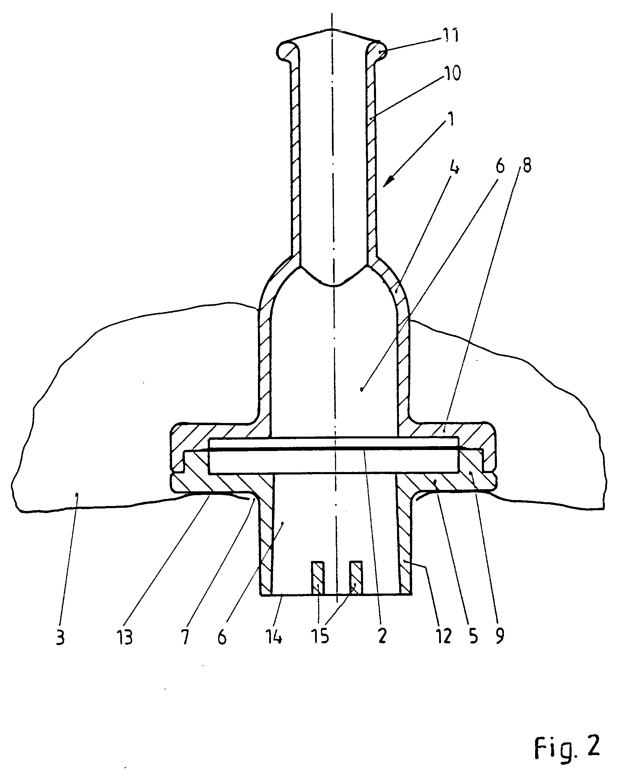

In Figur 2 ist eine zweite Ausführungsform der Vorrichtung dargestellt. Der Grundkörper 1 besteht auch hier aus den beiden Formteilen 4 und 5. Im Gegensatz zu der Ausführung gemäß Figur 1 ist der Zuschnitt 3 nicht in die Trennebene zwischen den beiden Formteilen 4 und 5 eingelegt, sondern nachträglich mit dem Formteil 5 durch eine Schweißung 13 unlösbar verbunden. Der Durchgangsquerschnitt 6 des Grundkörpers 1 ist patientenseitig im Bereich des Mundstückes 12, und zwar im Anschluß an das freie Ende 14 des Mundstückes 12 mit Stegen 15 versehen. Die Stege 15 können, wie dargestellt, parallel zueinander angeordnet sein oder auch gitterartig einen Schutz gegen das Eindringen von erbrochenen Speiseresten in den Durchgangsquerschnitt 6 bilden. Ebenso kann anstelle der Stege oder zusätzlich zu den Stegen hierfür ein grobmaschiges Flächengebilde als Vorfilter eingesetzt werden.

Bei der Ausführungsform der Vorrichtung gemäß Figur 3 ist neben dem Grundkörper 1, der ähnlich wie in Figur 1 dargestellt ausgebildet sein kann, noch ein Verlängerungskörper 16 vorgesehen, der ebenso wie der Grundkörper 1 rohrartig ausgebildet ist. Der Verlängerungskörper 16 weist einen Anschlußkonus 17 auf, der auf seiner Außenseite in Abstimmung auf die Innenseite des Mundstückes 12 konisch ausgebildet ist. Weiterhin ist am Verlängerungskörper 16 eine Mundplatte 18 ausgebildet, die eine ovale gekrümmte Form aufweist und damit der Mundpartie des menschlichen Kopfes angepaßt ist. Das patientenseitige Mundstück 12 der Vorrichtung ist hier auf der dem Anschlußkonus 17 abgekehrten Seite der Mundplatte 18 verwirklicht, so daß auch hier ein oder mehrere Stege 15 randseitig angeordnet sind bzw. ein Grobfilter integriert ist. Die Mundplatte 18 verhindert einerseits ein zu tiefes Einstecken des Mundstücks 12 des Verlängerungskörpers 16 in den Mund des Patienten und erbringt andererseits eine Abdichtung, damit die vom Helfer in den Patienten geschickte Luft diesen auch wirksam erreicht. Der Anschlußkonus 17 am Verlängerungskörper 16 und ein Anschlußkonus 19 am Formteil 5 stellen eine lösbare Befestigungseinrichtung zwischen dem Grundkörper 1 und dem Verlängerungskörper 16 dar. An dieser Stelle ist der Zuschnitt 3 mit seiner im Durchmesser entsprechend gestalteten Durchbrechung 7 lose eingefügt. Es ist aber auch möglich, den Zuschnitt 3 unlösbar zu befestigen. Dies geschieht vorteilhaft bei der Ausführungsform gemäß Figur 3 durch eine Schweißung an der Mundplatte. Sobald die Mund-zu-Mund-Beatmung beendet ist, kann der Grundkörper 1 von dem Verlängerungskörper 16 abgenommen werden. Der Verlängerungskörper 16 kann dabei mit dem Mundstück 12 im Munde des Patienten verbleiben, und es ist möglich, durch den Durchgangsquerschnitt 20 eine Intubation des Patienten durchzuführen.

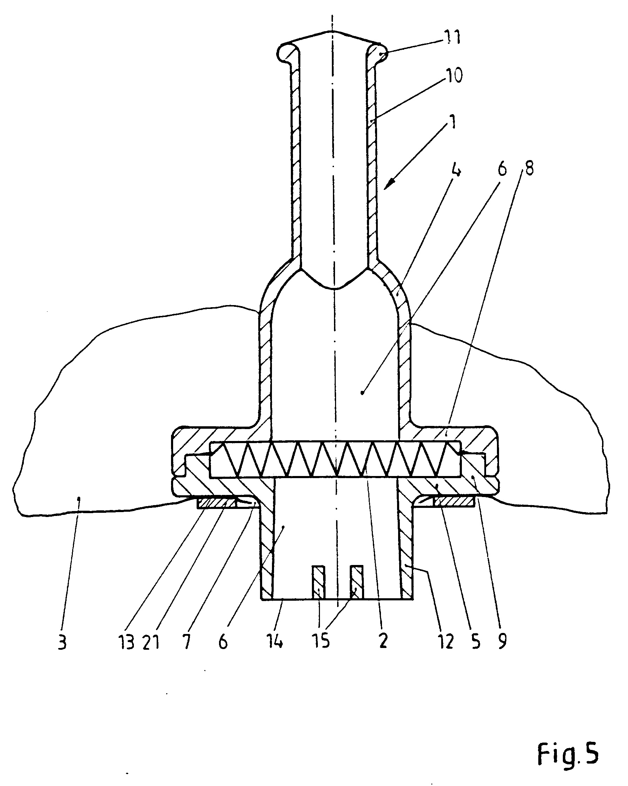

Bei der Ausführungsform gemäß Figur 5, die weitgehend mit derjenigen gemäß Figur 2 übereinstimmt, besitzt der Tiefenfilter 2 zickzackartige Formgebung, um eine möglichst große Fläche unterzubringen. Unter Umständen besteht dabei die Möglichkeit, auf die Anordnung der Erweiterungen 8 und 9 zu verzichten, so daß die Formteile 4 und 5 einen entsprechend kleineren Außendurchmesser aufweisen können. Der Zuschnitt 3 ist auch hier mit dem Formteil 5 verbunden. Er kann von einem Ring 21 aus Filz oder Schaumstoff abgedeckt sein, um eine Individualanpassung an den Mundbereich des Patienten zu ermöglichen.

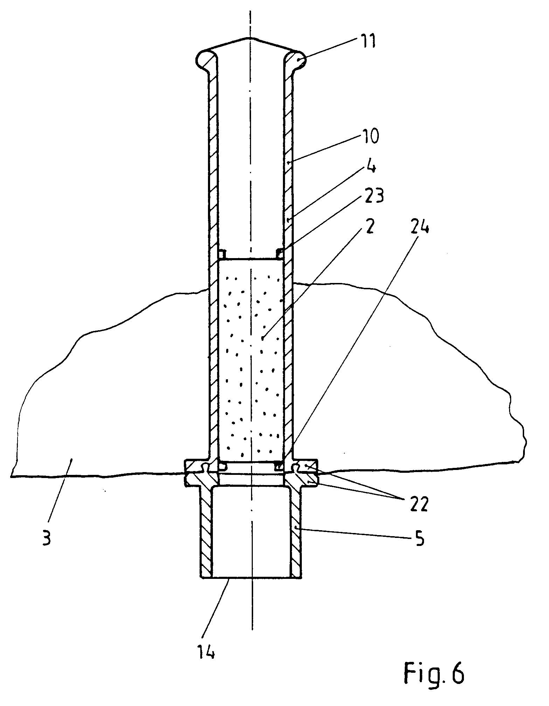

Bei der Ausführungsform gemäß Figur 6 sind die beiden Formteile 4 und 5 durch schnappverschlußartige Elemente 22 miteinander verbunden, wobei gleichzeitig der mit der Durchbrechung 7 versehene Zuschnitt 3 mit eingeklemmt und abgedichtet wird. Der hydrophobe Tiefenfilter 2 besitzt hier zylindrische, pfropfenartige Form und ist unter Reibschluß in das Formteil 4 eingesetzt, wobei Vorsprünge 23 und 24 den korrekten, unverrutschbaren Sitz garantieren. Der Tiefenfilter 2 kann hier aus gespritztem und gesintertem Kunststoff bestehen. Zum leichteren Einführen des Tiefenfilters 2 in das Formteil 4 kann dieses leicht konisch gestaltet sein. Diese Ausführungsform der Vorrichtung kann besonders preiswert hergestellt werden.

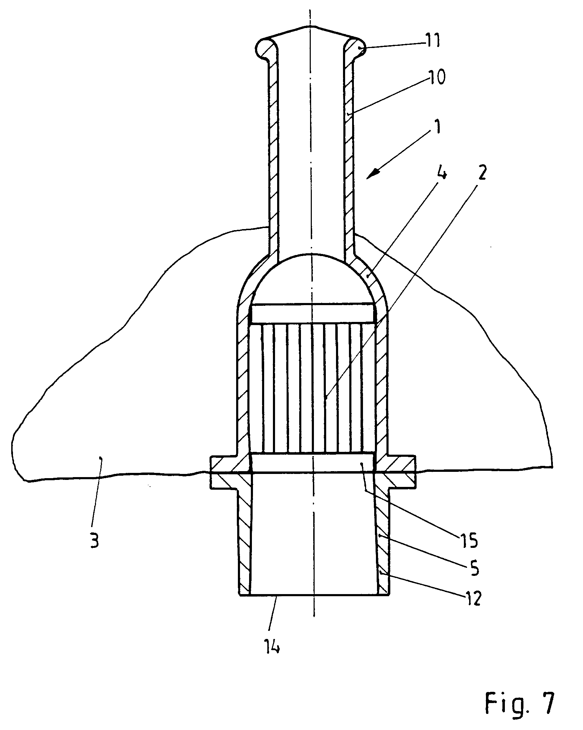

Die in Figur 7 dargestellte Ausführungsform der Vorrichtung weist in der Trennebene zwischen den Formteilen 4 und 5 die Stege 15 auf, die auch als Lochplatte oder Grobfilter ausgebildet sein können. Der Tiefenfilter 2 kann als Kerzenfilter, Faltenfilter, als zylindrischer Filterkörper o. dgl. ausgebildet sein.

### BEZUGSZEICHENLISTE:

- 1: - Grundkörper
- 2: - Tiefenfilter
- 3: - Zuschnitt
- 4: - Formteil
- 5: - Formteil
- 6: - Durchgangsquerschnitt
- 7: - Durchbrechung
- 8: - Erweiterung
- 9: - Erweiterung
- 10: - Mundstück
- 11: - Rand
- 12: - Mundstück
- 13: - Schweißung
- 14: - Ende
- 15: - Steg
- 16: - Verlängerungskörper
- 17: - Anschlußkonus
- 18: - Mundplatte
- 19: - Anschlußkonus
- 20: - Durchgangsquerschnitt
- 21: - Ring
- 22: - Elemente
- 23: - Vorsprünge
- 24: - Vorsprünge

## Patentansprüche

1. Vorrichtung für die Durchführung einer Mund-zu-Mund-Beatmung mit einem rohrartigen Grundkörper (1), der an seinen beiden Enden je ein Mundstück (10, 12) für den Helfer einerseits und den Patienten andererseits, sowie eine das Eindringen des Mundstücks (12) in den Mund des Patienten begrenzende Mundplatte (18, 9) aufweist und in dessen Durchgangsquerschnitt (6) ein hydrophober Tiefenfilter (2) angeordnet ist, dadurch gekennzeichnet, daß der rohrartige Grundkörper (1) mit einem sich im wesentlichen radial zu dem Grundkörper erstreckenden Zuschnitt (3) aus einem durchsichtigen Flächengebilde aus Kunststoffolie, textilem oder papierähnlichem Material versehen ist, daß der Zuschnitt (3) aus durchsichtigem Flächengebilde eine zumindest das Gesicht des Patienten überdeckende Größe aufweist, und daß das patientenseitige Mundstück (12) mit mindestens einem den freien Querschnitt (6, 20) durchsetzenden Steg (15) versehen ist.

2. Vorrichtung nach Anspruch 1, dadurch gekennzeichnet, daß der Zuschnitt (3) aus durchsichtigem Flächengebilde mit dem Grundkörper (1) unlösbar verbunden ist.

3. Vorrichtung nach Anspruch 2, dadurch gekennzeichnet, daß der Zuschnitt (3) aus durchsichtigem Flächengebilde mit dem Grundkörper (1) verklebt oder verschweißt ist.

4. Vorrichtung nach Anspruch 1, dadurch gekennzeichnet, daß der Zuschnitt (3) aus durchsichtigem Flächengebilde mit dem Grundkörper (1) lösbar verbunden ist.

5. Vorrichtung nach Anspruch 2 oder 4, dadurch gekennzeichnet, daß der Grundkörper (1) aus mehreren Teilen (4, 5) zusammengesetzt ist und daß der Zuschnitt (3) zwischen den Teilen des Grundkörpers (1) angeordnet ist.

6. Vorrichtung nach einem der Ansprüche 1 bis 5, dadurch gekennzeichnet, daß der Zuschnitt (3) aus durchsichtigem Flächengebilde eine an den Grundkörper (1) angepasste Durchbrechung (7) ausweist.

7. Vorrichtung nach einem der Ansprüche 1 bis 6, dadurch gekennzeichnet, daß ein rohrartiger Verlängerungskörper (16) vorgesehen ist, der eine sich radial und gekrümmt erstreckende Mundplatte (18) aufweist.

8. Vorrichtung nach Anspruch 7, dadurch gekennzeichnet, daß der Grundkörper (1) und der Verlängerungskörper (16) über einen Anschlußkonus (17, 19) miteinander verbunden sind.

9. Vorrichtung nach Anspruch 1, dadurch gekennzeichnet, daß der oder die Stege (15) als ein Grobfilter ausgebildet sind.

10. Vorrichtung nach Anspruch 1, dadurch gekennzeichnet, daß zusätzlich zu den Stegen (15) ein Grobfilter vor oder hinter den Stegen (15) vorgesehen ist.

11. Vorrichtung nach einem der Ansprüche 1 bis 10, dadurch gekennzeichnet, daß das durchsichtige Flächengebilde hydrophobe Eigenschaften aufweist.
